# EUROPEAN PATENT SPECIFICATION

(11) **EP 2 144 623 B1**
(45) Date of publication and mention of the grant of the patent: **25.01.2012**
(21) Application number: 08748096.8
(22) Date of filing: 02.04.2008
(51) Int. Cl.: A61K 38/18, A61P 17/06, A61P 29/00, A61P 37/06, C07K 1/30, C07K 14/495, A23J 1/20, A23C 21/08

(54) **PROCESS FOR THE MANUFACTURE OF A DAIRY-BASED ANTI-INFLAMMATORY COMPOSITION**
VERFAHREN ZUR HERSTELLUNG EINER ENTZÜNDUNGSHEMMENDEN ZUSAMMENSETZUNG AUF MILCHBASIS
PROCÉDÉ DE FABRICATION D'UNE COMPOSITION ANTI-INFLAMMATOIRE À BASE DE PRODUIT LAITIER

(30) Priority: 03.04.2007 US 909766 P
(43) Date of publication of application: 20.01.2010
(73) Proprietor: Advitech Inc., Québec, QC G2K 2C9 (CA)
(72) Inventor: JUNEAU, Christina, Québec G0A 4P0 (CA); DROUIN, Réjean, Québec, Québec G2C 1J1 (CA); MORONI, Olivier, Québec, Québec G2B 0A3 (CA)
(74) Representative: Delorme, Nicolas
(86) International application number: PCT/CA2008/000627
(87) International publication number: WO 2008/119185

(56) References cited:
- WO-A1-2007/038870
- US-B2- 7 141 262
- DROUIN R. ET AL.: 'XP-828L (Dermylex), a New Whey Protein Extract with Potential Benefit for Mild to Moderate Psoriasis' CAN. J. PHYSIOL. PHARMACOL. vol. 85, 2007, pages 943 - 951
- POULIN Y. ET AL.: 'Safety and Efficacy of a Milk-Derived Extract in the Treatment of Plaque Psoriasis: An Open-Label Study' JOURNAL OF CUTANEOUS MEDICINE AND SURGERY vol. 9, no. 6, 2005, pages 271 - 275, XP019432156

## Description

### BACKGROUND OF THE INVENTION

### a) Field of the invention

The present invention relates in general to a process for the preparation of a composition for the treatment of psoriasis and other inflammatory disorders.

### b) Description of the prior art

It is not known what causes psoriasis, although there is evidence of a genetic predisposition and an autoimmune etiology. Onset may be triggered by systemic infections such as streptococcus throat, skin injury, vaccinations, and certain oral medications such as steroids. Subsequently, the immune system is thought to induce inflammation and excessive skin cell reproduction, which can be exacerbated by additional factors such as stress and diet.

In normal skin, a cell moves from the basal layer through the granular layer in 4-5 weeks. In psoriatic lesions, the time is decreased 7-10 fold because of a shortened cell cycle time, an increase in the absolute number of cells capable of proliferating, and an increased rate of division. T cell mediated immune responses appear to be responsible for the inflammation and hyperproliferation of keratinocytes. Neutrophils are found in psoriatic lesions, associated with increased levels of plasminogen activators. Psoriatic fibroblasts have increased levels of enzymes involved in collagen synthesis, secondary to expansion of the papillary dermis. Psoriatic plaques comprise HLA-DR positive keratinocytes and Langerhans cells, and activated T cells expressing elevated levels of IL-2 receptors.

The typical lesion of psoriasis is a well-demarcated erythematous plaque, covered by thick, silvery scales. Psoriasis can become so extensive as to cause exfoliative erythroderma, in which the entire epidermal surface is in a state of hyperproliferation. Gluttate psoriasis is a form of the disease following streptococcal pharyngitis, with widely distributed characteristic 1-3 cm lesions. Pustular psoriasis is characterized by numerous sterile pustules of 2-5 mm in diameter, and may lead to an acute, explosive, life-threatening episode of fever, chills, leukocytosis, hypoalbuminemia, and hypocalcemia, demanding immediate, vigorous therapy. Previously stable plaque-type psoriasis can be acutely exacerbated by viral infections, particularly HIV. Psoriasis is also associated with five different forms of psoriatic arthritis, including distal interpharangeal involvement; an asymmetric, oligoarticular pattern; a symmetric polyarthritis; arthritis mutilans; and sacroiliitis and spondylitis.

The inflammation and hyperproliferation of psoriatic tissue is associated with a different histological and antigenic profile than normal skin. A panel of anti-carbohydrate monoclonal antibodies as described in the art, can be used to compare psoriatic tissue with the surrounding dermis. The glycosylation pattern in psoriatic epithelium is changed in two ways: some carbohydrates are expressed at an earlier stage of cell maturation; in addition, certain biosynthetic precursor antigens not expressed in normal skin are found in psoriatic skin.

Classical treatments of psoriasis include calcipotriene (a vitamin D₃ derivative), topical coal tar preparations, systemic antimitotic agents such as methotrexate, and retinoids, particularly etretinate.

Extensive psoriasis can be treated by photosensitization with oral 8-methoxypsoralen, followed by ultraviolet A. Corticosteroids are given for psoriatic arthritis and acute attacks of pustular psoriasis. More recently, cyclosporin A has been tested in clinical trials at doses of 3-7 mg/kg with promising results, but associated with the risk of renal toxicity.

Keratinocyte function is regulated via intracellular signaling pathways triggered by growth factors and adhesion molecules. Among them, the EGF family and the TGF-β family are thought to play central roles; they provide dual mode regulation of keratinocytes growth via the proliferation-stimulating effect of EGF and the proliferation-inhibiting effect of TGF-β. TGF-βs exert a wide range of biological effects on keratinocytes, such as growth inhibition, production of extracellular matrix, and synthesis of plasminogen activator and its inhibitor (PAl 1). Among them, growth inhibition is the most prominent.

TGF-β1 and TGF-β2 are synthesized and secreted in human keratinocytes. It seems that TGF-β3 is not a major TGF-β in human keratinocytes growth. Vitamin D3 is a strong inhibitor of keratinocyte growth. The involvement of TGF-β production induced by vitamin D3 increased the expression of TGF-β2 mRNA 4 to 5 fold. In contrast, TGF-β1 mRNA and TGF-β3 mRNA were not increased. Taken together, these data suggest that the intrinsic TGF-βs regulate autonomous growth of human keratinocytes, and have demonstrated that TGF-β antagonized the acanthotic and degenerative effect of TGF-α involved in psoriatic skin, but TGF-β alone did not cause granular layers to appear in the involved psoriatic epidermis, indicating that TGF-β alone cannot normalize the psoriatic condition of the epidermis.

TGF-β is now recognized as a potent growth inhibitor for human keratinocytes. TGF-β2, which is found through the normal human epidermis, is decreased in psoriatic epidermis. Since TGF-β is a strong growth inhibitor for human keratinocytes, this result indicates the possibility that the decrease of TGF-β2 is involved in the pathogenesis of psoriasis. Therefore, the decrease in TGF-β2 in psoriasis epidermis may induce the accelerated proliferation of keratinocytes and result in epidermal hyperplasia. TGF-β is also a potent immunosuppressive agent. So the decrease of TGF-β2 may permit propagation of an immune/inflammatory reaction in the dermis and epidermis of involved psoriasis. It has been supposed that an immune-activation triggers the growth activation of epidermal keratinocytes in psoriasis. In terms of growth regulation of keratinocytes, an involvement of two groups of growth factors and cytokines, proliferation-stimulating growth factors and proliferation-inhibitory growth factors, has been reported. EGF family growth factors (TGF-α, amphiregulin and HB-EGF), KGF and IL-6 are well known as proliferation-stimulating growth factors. TGF-β family growth factors are the major proliferation-inhibitory growth factors. An increase of TGF-α, amphiregulin and IL-6 is found in psoriasis. Autocrine- or cross-induction of EGF family growth factors may play an important role in epidermal hyperplasia.

One important group of growth factors involved in psoriasis mechanisms are the dermally-derived insulin-like growth factors (IGFs), which support keratinocyte proliferation. In particular, IGF-I and IGF-II are ubiquitous peptides each with potent mitogenic effects on a broad range of cells. Molecules of the IGF type are also known as "progression factors" promoting "competent" cells through DNA synthesis. The IGFs act through a common receptor known as the Type I or IGF-I receptor, which is tyrosine kinase linked. They are synthesized in mesenchymal tissues, including the dermis, and act on adjacent cells of mesodermal, endodermal or ectodermal origin. The regulation of their synthesis involves growth hormone (GH) in the liver, but is poorly defined in most tissues.

Particular proteins, referred to as IGF binding proteins (IGFBPs), appear to be involved in autocrine/ paracrine regulation of tissue IGF availability. Six IGFBPs have so far been identified. The exact effects of the IGFBPs is not clear and observed effects in vitro have been inhibitory or stimulatory depending on the experimental method employed. There is some evidence, however, that certain IGFBPs are involved in targeting IGF-I to its cell surface receptor.

Skin, comprising epidermis and underlying dermis, has GH receptors on dermal fibroblasts. Fibroblasts synthesize IGF-I as well as IGFBPs-3, -4, -5 and -6, which may be involved in targeting IGF-I to adjacent cells as well as to the overlaying epidermis. The major epidermal cell type, the keratinocyte, does not synthesize IGF-I, but possesses IGF-I receptors and is responsive to IGF-I.

Novel biotechnology approaches to psoriasis treatment relate to such growth factors, in particular TGF-β and/or IGF produced recombinantly or by isolation/enrichment from dairy industry by-products.

For example, WO 01/025276 filed by Campina Melkune B.V. and N.V. Nutricia discloses a process for the extraction of TGF-β and IGF-1 from milk products. The process described proposes different purification steps including a cation exchange chromatography (CEC) followed by passage through a hydroxyapatite column. The fractions resulting from this purification step contain IGF-1 substantially absent of TGF-β and a fraction containing TGF-β substantially absent of IGF-1. These purified fractions obtained by different chromatography steps contrast with the present invention in which physical separation techniques give a composition mainly composed of whey protein (β-lactoglobulin and α-lactalbumin) enriched in both TGF-β and IGF-1.

WO 03/008447 filed by Campina B.V. and Numico Research B.V. (equivalent to US 2004/0219225) disclose processes closely related with the one disclosed in WO 01/025276 for the separation of TGF-β and IGF-1 from milk products. This process consists of applying a milk product to an ion exchange chromatography (IEC) followed by hydrophobic interaction chromatography (HIC) in order to obtain different purified fractions described as: a purified TGF-β fraction (containing between 1400-2500 µg of TGF-β per gram of protein while containing less than 8 µg of IGF-1 per gram of protein); a purified IGF-1 fraction (containing between 150-180 µg of IGF-1 per gram of protein while containing less than 30 µg of TGF-β per gram of protein); a purified lactoperoxydase fraction; and a purified immunoglobulin fraction. This fractionation process differs from the present invention as the IEC and HIC allow for the separation of particular compound from milk protein while the physical separation of the present invention allow the enrichment of particular growth factor in a product where the overall composition in mainly composed of major whey proteins (β-lactoglobulin, α-lactalbumin, etc..). This major difference in the process strategy is also reflected in the final concentrations of TGF-β in the product issued from the process proposed in the present invention.

WO 2007/012748 filed by Compagnie Laitière Européenne also discloses a process for obtaining a milk derived protein composition enriched in TGF-β. This process uses a IEC step followed by ultrafiltration and diafiltration in order to concentrate the product obtained by the IEC step. This process and the composition obtained contrast with the present invention. In fact, the process of the present invention propose acid precipitation of whey protein and separation of the precipitate by physical means (like centrifugation) in order to obtain a composition principally composed of β-lactoglobulin and α-lactalbumin. In opposition, the composition obtained with the process describe in WO 2007/012748 consist principally in lactoperoxydase and immunoglobulin.

Finally US patent 7,141,262 issued to Maubois et al. discloses a method for obtaining a TGF-β enriched protein fraction in activated form. However, in contrast to the present invention, this document discloses a method and a composition that contains a majority (45 to 80%) of α-lactalbumin and which intends to limit the amount of β-lactoglobulin as much as possible (less than 11 %). Considering the state of the art described above, there is still a significant need for a cheap, efficacious and reproducible process for the production of a psoriasis treatment that avoids the disadvantages associated with the currently available topical or systemic treatments.

### SUMMARY OF THE INVENTION

One particular aspect of the present invention is to provide a process for the isolation or enrichment of growth factors carried out on starting material which is commercially available and called whey proteins (WP) defined as the proteins appearing in the supernatant of milk after precipitation at pH 4.6 or after rennet precipitation. Whey proteins can present themselves in the form of a whey protein isolate (WPI), a whey protein concentrate or other whey protein derivatives.

The process is generally defined as comprising the following steps:
a) rehydration of whey proteins to a concentration of between 2% and 10% (w/v);
b) acid precipitation of the solution at a pH of between 4.5 and 6;
c) recovery of the precipitate; and
d) neutralization to about pH 7.
e) Optionally, the recovered material is subjected to a short pasteurization (for example about 15 seconds at about 75°C).
f) Optionally, the retentate is ultrafiltrated.
g) Furthermore, the retentate may be spray-dried to obtain a powder that will be used "as such" or in admixture with a physiologically acceptable excipient for *in vitro* or *in vivo* use.

For the purpose of the present invention the following terms are defined below.

The terms "about" and "around" as used herein refer to a margin of + or -10% of the number indicated.

The term "psoriatic tissue" refers to tissue affected by psoriasis and affected cells contained within the tissue, but not to cells that have migrated to the site such as leukocytes. Preferably, the psoriatic tissue is from a human.

The expression "effective amount" as used herein is intended to mean an amount sufficient to effect a beneficial or desired clinical result. An effective amount can be administered in one or more doses. For purposes of this invention, an effective amount of growth factors and/or dairy derived proteins, or other composition is an amount that induces a treatment or prophylactic response against at least one psoriasis responsible factor.

The terms "polypeptide", "peptide" and "protein" are used interchangeably to refer to polymers of amino acids of any length, and may be interrupted by non-amino acids.

The terms "individual" or "subject" treated according to this invention is a vertebrate, preferably a mammal, more preferably a human. Mammals include, but are not limited to, farm animals, sport animals, rodents, primates, and pets.

The terms "minerals" or "ashes" as used herein are intended to mean salt constituents or minerals at concentrations normally found in dairy products. For example, there can be different concentration of calcium, phosphorus, magnesium, potassium, sodium, chloride, sulfur, and citric acid. This also means that trace elements known in the art as found in dairy products can be comprised or inferred therein.

Other terms used in this disclosure are explained where they arise.

### BRIEF DESCRIPTION OF THE DRAWINGS

Fig. 1 shows a dose-dependent inhibition of Concanavalin A (ConA)-induced murine splenocytes proliferation by the composition of the present invention (XP-828L) as described in detail in example IV;
Fig. 2 illustrates the effect of the composition of the present invention on CD8⁺ proportion in ConA-activated mice splenocytes. Cells were incubated for 72h in the presence of 1.25 µg/ml of ConA. Data are expressed as means ± SD as described in detail in example IV;
Fig. 3 shows a dose-dependent inhibition of ConA-induced murine splenocytes IL-2 production **(A)** and IFN-γ production **(B)** by the composition produced in example I and described in example II with the experimental details as described in example IV;
Fig. 4 illustrates the effect of colorectal administrations of the composition produced in example I (at a dose of 78.2 mg/kg = 1x) and described in example II on daily body weight variations of Sprague-Dawley rats treated with 2,4,6-trinitrobenzenesulfonic acid (TNBS), a chemical inflammatory inducer, as described in detail in example V;
Fig. 5 illustrates the effects of colorectal administrations of the composition produced in example I and described in example II on weight-to-length ratios of the colons of Sprague-Dawley rats treated with TNBS, as described in detail in example V;
Fig. 6 illustrates the effects of colorectal administrations of the composition produced in example I and described in example II on macroscopic damages of rat colon tissues previously irritated with TNBS, as described in detail in example V;
Fig. 7 illustrates the effects of the composition produced in example I and described in example II on the macroscopic appearance of lesions in TNBS colitis, as described in detail in example V;
Fig. 8 shows hematocrit levels before and during oral treatment of HLA-B27 transgenic rats with different doses of the composition produced in example I and described in example II, where **A** is a placebo, **B** is the composition (7.82 mg/kg), **C** is the composition (78.2 mg/kg), and D is the composition (782 mg/kg), as described in detail in example VI;
Fig. 9 shows hemoglobin levels before and during oral treatment of HLA-B27 transgenic rats with different doses of the composition produced in example I and described in example II, where **A** is a placebo, **B** is the composition (7.82 mg/kg), **C** is the composition (78.2 mg/kg), and **D** is the composition (782 mg/kg) as described in detail in example VI; and
Fig. 10 shows the relative improvements or deterioration of primary and secondary endpoints from the double-blind, placebo-controlled study of Example VII. The vast majority of the patients taking the composition improved all psoriasis assessment parameters from baseline at day 56 and day 112, indicating that patients could certainly benefit from further improvement with longer use of the present composition compared to a placebo; (•)XP-828L and (•) placebo.

### DETAILED DESCRIPTION OF PARTICULAR EMBODIMENTS

The present invention now will be described more fully hereinafter with reference to the accompanying figures. This invention may be embodied in many different forms and should not be construed as limited to the embodiments set forth herein; rather, these embodiments are provided so that this disclosure will be thorough and complete, and will fully convey the scope of the invention to those skilled in the art.

The process for the production of anti-psoriatic composition as defined hereinbelow is generally defined as comprising the steps as presented hereinabove.

Particularly the following steps are carried out:

In step a) the whey proteins are rehydrated to a concentration of between 2% and 10% (w/v) in water; particularly to a concentration of between 3% and 8%; more particularly around 5% in water.

Particularly, in step a) the hydration is carried out at a temperature of about 4°C to about 10°C.

In step b) acid precipitation of the solution is carried out at a pH of between 4.5 to 6.0; particularly between 4.6 and 5.8; more particularly between 4.8 and 5.2, most particularly around 5.1.

Particularly, in step b), the precipitation is carried out at a temperature of between 10°C and 60°C; particularly between 20°C and 50°C; and more particularly between 30°C and 40°C.

In step c) recovery of the precipitate is carried out by physical means such as, for example: settling, sedimentation, centrifugation, filtration, aggregation and dialysis; preferably by centrifugation.

Particularly, in step c), recovery of the precipitate is carried out at a temperature of between 10°C and 60°C; particularly between 20°C and 50°C, more particularly between 30°C and 40°C.

In step d) neutralization to pH between 6.7 to 7.3; particularly between 6.8 and 7.2; more particularly at a pH around 7 is carried out.

Optionally in step e) the recovered material may be subjected to a short pasteurization of less than 2 minutes, preferably less than 1 minute, more preferably less than 30 seconds at a temperature of between 70°C and 80°C, preferably between 72°C and 78°C, more preferably between 74°C and 76°C; most preferably at about 75°C.

Further optionally in step f) the retentate may ultrafiltrated on membranes having molecular weight cut-off (MWCO) up to a maximum of 10 kDa.

Still optionally in step g) the retentate may be spray-dried to obtain a powder that will be used "as such" or in admixture with one or more physiologically acceptable excipient(s) for *in vitro* or *in vivo* use.

Specifically, the process is particularly defined as comprising the following steps:
a) rehydration of whey proteins to a concentration of about 5% (50 g/L) in water;
b) acid precipitation of the solution at a pH of about 5.1 and a temperature of about 35°C;
c) recovery of the precipitate by centrifugation at a temperature of about 35°C; and
d) neutralization to about pH 7.
e) Optionally, the recovered material is subjected to a short pasteurization (for example about 15 seconds at about 75°C).
f) Optionally, the retentate is ultrafiltrated.

In accordance with a particular aspect of the invention, there is provided composition and methods for treating psoriasis in an individual, comprising administering a composition effective in stimulating a specific immunological response against a physiological imbalance aberrantly expressed in psoriatic tissue. These composition(s) comprise a cocktail of products that shares growth and regeneration characteristics of a growth factor that is aberrantly expressed in psoriatic tissue (such as human psoriatic tissue). Particular growth factors included in the composition of the present invention, but not limited thereto are transforming growth factors, such as TGF-β1 and TGF-β2, and at least 50% of dairy derived proteins. Preferably, the composition of the present invention will be comprised of more than 80% of dairy derived proteins, and most preferably more than 85% of dairy derived proteins. These dairy-derived proteins may be comprised, for example but not limited to, of between 30 to 85%(w/w) of β-lactoglobutin, preferably between 50 and 70% and most preferably at least 60%; and between 0 and 25% (w/w) of α-lactalbumin, more preferably between 5 and 15%, most preferably between 5 and 8%; and between 0 to 15% (w/w) of lactoferrin, preferably between 0 to 8%, more preferably between 4 to 5 %.

Milk proteins are naturally present as follows:

| | grams/ liter | | % of total protein | |
|---|---|---|---|---|
| Total Protein | | 33 | | 100 |
| Total Caseins | | 26 | | 79.5 |
| alpha s1 | 10 | | 30.6 | |
| alpha s2 | 2.6 | | 8.0 | |
| beta | 9.3 | | 28.4 | |
| kappa | 3.3 | | 10.1 | |
| Total Whey Proteins | 6.3 | | 19.3 | |
| alpha lactalbumin | 1.2 | | 3.7 | |
| beta lactoglobulin | 3.2 | | 9.8 | |
| BSA | 0.4 | | 1.2 | |
| Immunoglobulins | 0.7 | | 2.1 | |
| Proteose peptone | 0.8 | | 2.4 | |

In general, the composition of the present invention typically comprises:

| **Protein** | **Typical values** | **Window** |
|---|---|---|
| TGF-β2 | 11.5 µg/g | 8 µg/g to 20 µg/g |
| TGF-β1 | 0.3 µg/g | 0.01 to 5 µg/g |
| IGF-1 | 0.1 µg/g | 0.01 to 2 µg/g |
| β-lactoglobulin (β-Lg) | 70% | 30% to 85% |
| α-lactalbumin (α-Lac) | 6% | 0% to 25% |
| Lactoferrin (Lf) | 4,5% | 0% to 15% |
| Immunoglobulin (IgG) | 5,5% | 2% to 15% |
| Hydrosolubility | 60% | Over 30% |
| Fat | 1% | 0% et 10% |

Whey Proteins: The proteins appearing in the supernatant of milk after precipitation at pH 4.6 or after rennet precipitation are collectively called whey proteins. These globular proteins are more water soluble than caseins and are subject to heat denaturation. Native whey proteins have good gelling and whipping properties. Denaturation increases their water holding capacity. The main fractions are β-lactoglobulin, alpha-lactalbumin, bovine serum albumin (BSA), lactoferrin (Lf) and immunoglobulins (Ig).
β -Lactoglobulins: (MW - 18,000; 162 residues) This group, including eight genetic variants, comprises approximately half the total whey proteins. β -Lactoglobulin has two internal disulfide bonds and one free thiol group. The conformation includes considerable secondary structure and exists naturally as a non-covalent linked dimer. At the isoelectric point (pH 3.5 to 5.2), the dimers are further associated to octamers but at pH below 3.4, they are dissociated to monomers.
alpha-Lactalbumins: (MW - 14,000; 123 residues) These proteins contain eight cysteine groups, all involved in internal disulfide bonds, and four tryptophan residues. alpha-Lactalbumin has a highly ordered secondary structure, and a compact, spherical tertiary structure. Thermal denaturation and pH <4.0 result in the release of bound calcium.
Lactoferrin: Lactoferrin is a glycoprotein that belongs to the iron transporter or transferrin family. Lactoferrin belongs to the transferrin family proteins (TF, melanotransferrin, ovotransferrin, etc.). Its molecular weight is 80,000 Da (80 kDa. In addition to its presence in milk, it is also found in exocrine secretions of mammals and is released from neutrophil granules during inflammation. Lactoferrin (LF) is a globular multifunctional protein with antimicrobial activity (bacteriocide, fungicide) and is part of the innate defense, mainly in mucosa. Lactoferrin is found in milk and many mucosal secretions such as tears and saliva. Human colustrum has the highest concentration, followed by human milk, then cow milk. The isoelectric point of both human breast milk lactoferrin and human granulocyte lactoferrin is 5.5-6.2.

While a detectable immunological or tissue response is likely to be beneficial, efficacy can also be deduced by an improvement in symptoms or control of the psoriatic condition beyond what would be expected without treatment.

The active composition as produced according to the process of the invention is distinguished by strong anti-inflammatory action.

For prophylaxis, the composition is administered to a patient diagnosed with psoriasis, and prone to frequent relapse, in order to decrease manifestations of the disease in frequency and strength. Treatment in the manifest stage leads to its curtailment and to the alleviation of the symptoms.

In all types of psoriasis, the composition of the present composition can be used prophylactically and for the treatment of the disorders.

Estimates of the prevalence of psoriasis vary from 0,5 to 6%, with rates varying between countries and races. Different types of psoriasis can be prevented or treated with the composition of the present invention. Plaque-type psoriasis is the most common form of the disease, occurring in more than 80% of cases. Guttate psoriasis occurs in about 10% of patients with psoriasis, and erythrodermic and pustular psoriasis each occur in fewer than 3% of patients. Nail psoriasis is generally the first sign of disease in 4% of patients. Between 5 to 10% of patients with psoriasis have psoriatic arthritis, a destructive and occasionally disabling joint disease. The course of psoriatic arthritis varies, with some having mild changes and others severe, rapid destruction of joints. Skin problems such as dryness or lesions are symptoms associated with psoriasis.

Erythrodermic psoriasis is characterized by generalized inflamed erythema and widespread scaling, affecting up to 100% of the body surface area. Patients lose many of the protective functions of the skin, including the skin's ability to protect against infection, control body temperature, and prevent loss of fluids and nutrients through the cutaneous surface.

Generalized pustules psoriasis is characterized by development of sterile pustules covering large portions of the trunk and extremities. In severe cases, pustules become confluent forming large areas of pus. In this case also, many of the skin's protective functions are lost, making patients susceptible to infection and loss of fluids and nutrients.

A further neurosensory phenomenon is to be regarded as itching in the case of atopic skin, and also itching in the case of psoriasis related disorders.

According to the invention, it is therefore possible to make available the composition as produced by the process of the invention which, in particular, prevent neurosensory phenomena or alleviate them or rapidly make them fade, i.e. are suitable for prophylaxis and/or treatment.

"Stinging" phenomena can be regarded as disorders to be treated cosmetically or orally, or in other cases by other ways of administration. Severe itching, however, in particular in the case of atopy, in particular neurodermatitis and severe itching of the skin occurring during psoriasis, can also be regarded as a relatively serious dermatological disorder.

The composition as produced according to the invention can in particular also be used on skin superficially appearing to be healthy, e.g. in the case of psoriasis and atopy, i.e. also in addition to the diseased skin areas and, in particular, here too in the case of related psoriasis disorders.

The composition as produced according to the invention can also be incorporated into customary oral, pharmaceutical, dermatological, and cosmetic bases for preferred oral administrations and the corresponding pharmaceutical, in particular dermatological, and cosmetic topical preparations or compositions can thus be obtained. The concentration of compounds in the composition of the present invention are adjusted depending on the needs. TGF-β1 can be found at concentration of between 0.01 to 5 µg per gram of composition and TGF-β2 can be found at concentration of between 5 to 50 µg per gram of composition. Preferably, TGF-β1 is found at concentration of 0.1 to 5 µg/g, and TGF-β2 at between 8 to 20 µg/g of composition. More preferably, TGF-β1 is found at concentration of 0.2 to 1.2 µg/g, and TGF-β2 at between 10 to 18 µg/g of composition. The preparations can be used daily in a customary manner. Preferably, the composition also comprises Insulin-like Growth Factor-1 (IGF-1) wherein the concentration of IGF-1 ranges from 0.01 to 2, preferably between 0.05 to 1 µg/g of composition.

In another embodiment of the present invention, the composition comprises at least 70% (w/w) of dairy derived proteins. The total concentration of proteins in the composition would preferably be of at least 80% wherein at least 60% of this protein content is β-lactoglobulin, preferably between 60% and 75%. Most preferably between 65% and 70% of total proteins is comprised of β-lactoglobulin. Dairy proteins or dairy products as used herein may include milk, colostrums, or whey proteins or derived products or fractions thereof.

The present invention also addresses the underlying T-cell disorder that results in an inflammatory condition such as one due to psoriasis. The present inventors have recognized that most, if not all, of the current therapies for inflammatory disorders such as psoriasis or similar T-cell mediated inflammatory skin conditions are designed to modulate T-cell activity and thereby improve symptoms of inflammation. It is possible that a major problem with the current treatments is that the therapy itself is so toxic that it may promote recurrence during healing. The toxicity of current treatments unleashes some or all of the cytokines that are associated with the promulgation of these chronic and often rebounding skin diseases.

The synergistic effects of the two TGF-β factors in activated form as produced according to the process of the invention and other dairy-derived proteins result in a non-toxic, highly effective treatment for inflammatory disorders, for example psoriasis, that is without the side effects observed with virtually all other therapies for moderate to severe psoriasis (mild psoriasis may be successfully treated with proper moisturizing). In addition, and as illustrated by the following studies, the most common over-the-counter treatment for psoriasis, namely coal tar, may be made more effective by the use of the present composition.

The active compounds according to the present invention can be mixed with customary pharmaceutically acceptable excipients or vehicles and, if appropriate, with other auxiliaries and administered, for example, orally or parenterally. They can preferably be administered orally in the form of granules, capsules, pills, tablets, film-coated tablets, sugar-coated tablets, syrups, emulsions, suspensions, dispersions, aerosols and solutions and also liquids, or else also as suppositories, vaginal suppositories or parenterally, e.g. in the form of solutions, emulsions or suspensions. Preparations to be administered orally can contain one or more additives such as sweeteners, aromatizing agents, colorants and preservatives. Tablets can contain the active compound mixed with customary pharmaceutically tolerable auxiliaries, for example inert diluents such as calcium carbonate, sodium carbonate, lactose and talc, granulating agents and agents which promote the disintegration of the tablets on oral administration, such as starch or alginic acid, binding agents such as starch or gelatin, lubricants such as magnesium stearate, stearic acid and talc.

Physiologically acceptable excipients are, for example, lactose, sorbitol, gelatin, maize starch, stearic acid, ethanol, propylene glycol, polyethylene glycol, ethers of tetrahydrofurfuryl alcohol, microcrystalline cellulose, povidone, croscarmellose sodium, colloidal silicon dioxide, magnesium stearate, hydroxypropyl cellulose, and water.

The formulations are prepared, for example, by extending the active compounds with solvents and/or excipients, if appropriate using emulsifiers and/or dispersants, it being possible, for example, in the case of the use of water as a diluent optionally to use organic solvents as auxiliary solvents.

Administration is carried out in a customary manner, preferably orally or parenterally, in particular perlingually, sublingually, or intravenously. In the case of oral administration, apart from the excipients mentioned, tablets, of course, can also contain additives, such as sodium citrate, calcium carbonate and dicalcium phosphate together with various additives, such as starch, preferably potato starch, gelatin and the like. Furthermore, lubricants such as magnesium stearate, sodium lauryl sulphate and talc can additionally be used for tableting. In the case of aqueous suspensions and/or elixirs, which are intended for oral administration, the active compounds can be mixed, apart from with the above-mentioned auxiliaries, with various flavors enhancers or colorants.

In the case of parenteral administration, solutions of the active compounds using suitable liquid excipients can be employed.

Capsules can contain the active compound as a single constituent or mixed with a solid diluent such as calcium carbonate, calcium phosphate or kaolin. The injectable preparations are also formulated in a manner known per se.

Ointments and topical formulations are particularly of interest also when considering the use of the composition of the present invention for treating psoriasis.

The preferred compositions according to the invention can be formulated as liquid, pasty or solid preparations, for example as aqueous or alcoholic solutions, aqueous suspensions, emulsions, for example W/O (water/oil) or O/W (oil/water) emulsions, ointments, gels, lotions, creams, oils, powders or sticks. Depending on the desired formulation, the active compounds can be incorporated into pharmaceutical and cosmetic bases for topical application, which as further components contain, for example, oil components, fat and waxes, emulsifiers, anionic, cationic, ampholytic, zwitterionic and/or non-ionic surfactants, lower mono- and polyhydric alcohols, water, preservatives, buffer substances, thickeners, fragrances, colorants and opacifiers. Preferably, the emulsions, e.g. W/O emulsions, or ointments are used.

Furthermore, it is preferred according to the invention to add antioxidants to the active compounds and to the pharmaceutical and topical preparations. The use of natural or naturally identical compounds such as, for example, tocopherols is particularly preferred here. The antioxidants mentioned are contained in the compositions according to the invention, for example, in amounts from 0.01-5% by weight, in particular from 0.5-2% by weight, based on the total composition.

The present invention will be more readily understood by referring to the following examples which are given to illustrate the invention rather than to limit its scope.

### EXAMPLE I

### Preparation of composition

1000 kg of a whey protein isolate was rehydrated up to 5% (50 g/L) in deionized water and stabilized overnight at 4°C under agitation. The pH of the solution was adjusted to between 4.8 and 5.2 by addition of concentrated HCl. This adjustment induced protein precipitation. The precipitate was then recovered by centrifugation on a Westfalia separator at 11 m³ per hour and washed with acidified water. The precipitate was rehydrated to obtain 800 liters, which was thermally treated at 75°C during 15 seconds, before concentration and spray drying. Approximately 60 kg of composition was obtained.

### EXAMPLE II

### Analysis of the composition

Typical content of the composition (XP-828L) is presented in Table 1. Proteins were analyzed by the Kjeldahl Method and Dye Binding Method (AOAC Official Method 930.29; AOAC Official Methods of Analysis (1995)), while fat content was quantified by the Roese-Gottlieb Method (AOAC Official Method 932.06), humidity by drying in a dry oven, betalactoglobulin and α-lactalbumin by RP-HPLC and growth factors by ELISA methods.

**Table 1: Typical analysis of the composition**

| Compound | Quantity (w/w) |
|---|---|
| Proteins (hydro-soluble proteins) | 90% (48%) |
| α-lactalbumin | 6% |
| β-lactoglobulin | 66% of total proteins |
| IGF-1 | 0.1 µg/g of the composition |
| TGF-β2 | 11.7 µg/g of the composition |
| TGF-β1 | 0.3 µg/g of the composition |
| Fat | 1.9% |
| Humidity | 4.2% |
| Ashes | 2% |

### EXAMPLE III

### Typical analysis of XP-828L

TGF-β2 : Concentration of 11.5 µg/g of powder.

Typical inhibition of murine splenocyte proliferation:
1 µg/ml = inhibition from -5 to10%
10 µg/ml = inhibition from 5 to 20%
100 µg/ml = inhibition from 20 to 45%
1000 µg/ml = inhibition from 45 to 75%

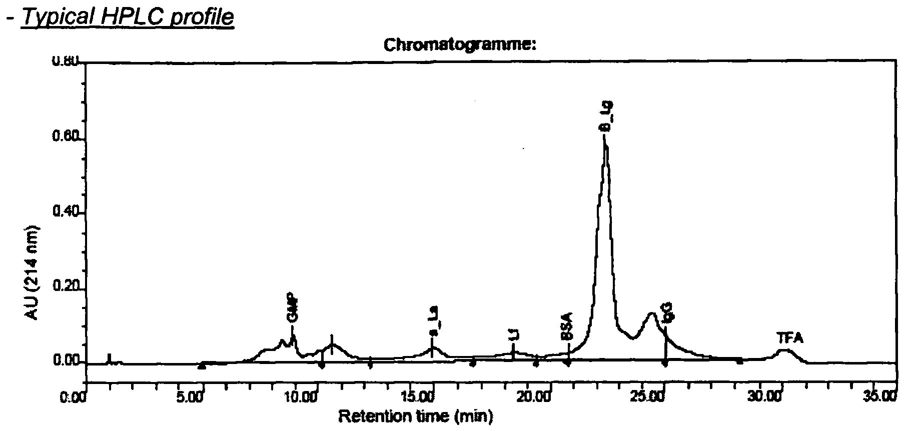

### EXAMPLE IV

### Inhibition on proliferation of mice splenocytes

TGF-β has multiple immunosuppressive effects at the cellular level and has been described as inhibiting type 1 and type 2 cells, B cells, T cells, macrophages and natural killer cells. TGF-β blocks cell-cycle progression and may have a direct effect on expression of the gene encoding IL-2. Importantly, it can suppress the expression of IL-12 and IL-2 receptors, as well as down-regulate MHC (major histocompatibility complex) class II expression on macrophages (specifically by antagonizing TNF-α and IFN-γ). The importance of TGF-β in the induction and maintenance of normal tolerance has been illustrated by TGF-β-knockout mice, who incur severe, spontaneous, multi-organ, autoimmune disease associated with autoantibody production, which proves fatal in early life. T lymphocytes isolated from murine spleen (splenocytes) are immune cells that respond to ConA (mitogenic stress) by proliferation and cytokines production. The purpose of this example was to evaluate the inhibition effect of the composition produced in example I and described in example II on splenocytes proliferation and production of pro-inflammatory cytokines, and try to elucidate some aspects of the composition's mechanism of action.

Female BALB/c mice, 6-8 weeks old, were obtained from Charles River (Montreal, Canada). Mice were sacrificed by CO₂ inhalation and single-cell suspensions were prepared individually from murine spleen under aseptic conditions. Murine lymphocytes (1.25 x 10⁶ cells/ml) were treated with ConA (1.25 µg/ml) for 72 hours. Alamar blue® was added 24 hours before measuring ConA-induced lymphocytes proliferation. Cytokines (IL-2 & IFN-γ) levels were also determined in the supernatant following the incubation period. BSA was used as a protein control (1-1000 µg/ml).

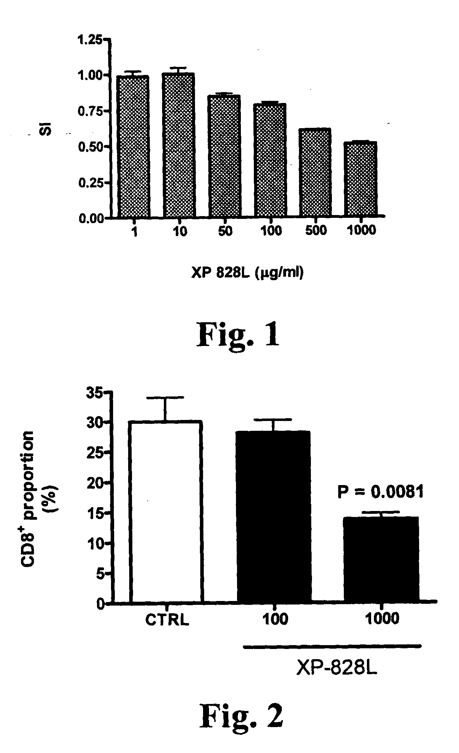

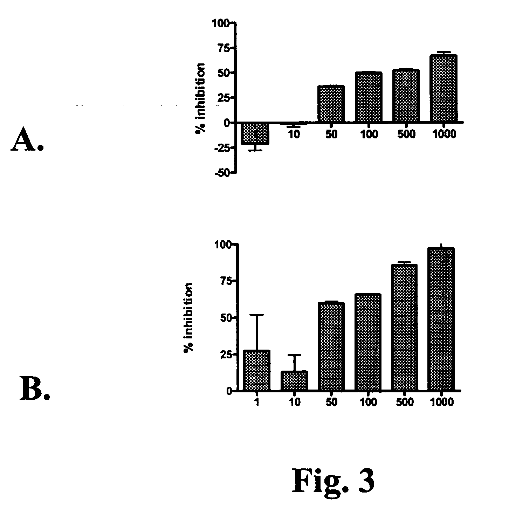

The composition produced in example I and described in example II (1, 10, 50, 100, 500, and 1000 µg of the total composition per ml of culture medium), was added to the cells for the whole incubation period and dose-dependently decreased 1) the lymphocyte proliferation (Fig. 1) the proportion of CD8 lymphocytes (Fig. 2), and the production of IL-2 and IFN-γ (Fig. 3). As shown in Fig. 1, there was a significant inhibition of induced proliferation by the composition. Without wishing to be bound to theory, these results suggest that the composition presents immunomodulatory functions and that it can be used to address immune-mediated chronic inflammatory diseases such as psoriasis, inflammatory bowel diseases and related arthropathies.

### EXAMPLE V

### Test on TNBS-induced inflammation in healthy rats

Inflammatory bowel diseases (IBD) is a term used to describe a collection of diseases that involve the intestine and are characterized by chronic inflammation, sometimes accompanied by ulceration in the small or large intestine. Cytokines play an important role in modulating the immune system since they are rapidly synthesized and secreted from activated inflammatory cells and induce the production of adhesion molecules and other inflammatory mediators, such as reactive oxygen metabolites, nitric oxide, and lipid mediators. Cytokines induce, amplify, prolong, and inhibit inflammation. Recently, several murine models of colitis have been developed which have highlighted the important role that abnormalities of the immune system, particularly those affecting T cells, may play in disease pathogenesis. The purpose of this example was to characterize the effect of local application of the composition produced in example I and described in example II on TNBS-induced colitis in rats.

Male Sprague Dawley rats (Charles River Laboratories, Montreal), were kept in individual cages, at 20°C and 55% relative humidity with 12h light/dark cycles, in a facility meeting the Canadian Council on Animal Care guidelines. The rats were subjected to a 24-hour starving period prior to inflammatory stress induction. Colonic inflammation was induced by using the following technique: briefly, the rats were lightly anesthetized with isoflurane after overnight food deprivation, and a polyethylene catheter was inserted 8 cm into the colon via the anus. TNBS (Sigma-Aldrich, Canada) dissolved in 50% (vol/vol) aqueous ethanol (25mg/ml) was injected into the colon (total volume of 1 ml/rat). The control rats received 1 ml of PBS intracolonically in place of TNBS and ethanol.

Three groups of rats were treated in the following manner:
Group 1: Non-stressed control receiving 2 ml of PBS (n=2) once a day throughout the protocol;
Group 2: Stressed control: TNBS (stress agent) in the intestinal lumen receiving 2 ml of PBS once a day, 5 days prior to administration of the stress agent in the lumen and 24 hours following the administration of the stress agent in the lumen (n=8);
Group 3: Colorectal administration of the composition produced in example I and described in example II (78.2 mg/kg in 2 ml of PBS, n=7) once a day, 5 days prior to administration of the stress agent in the lumen and 24 hours following the administration of the stress agent in the lumen.

Animals were weighed every day for the entire time of the protocol. Forty-eight hours following the TNBS administration, the rats were sacrificed and a 1 cm excision of the intestine was sampled. Samples were weighed and macroscopic colonic damage was scored by the following scale:
0. No damage
1. Localized hyperemia, no ulcer
2. Ulcerations without hyperemia of bowel wall thickening
3. Ulceration with inflammation at one site
4. Two or more sites of ulceration/inflammation
5. Major sites of damage extending more than 1 cm along the length of colon
6-10. If damage extends more than 2 cm along length of colon, score is increased by one for each additional 1 cm

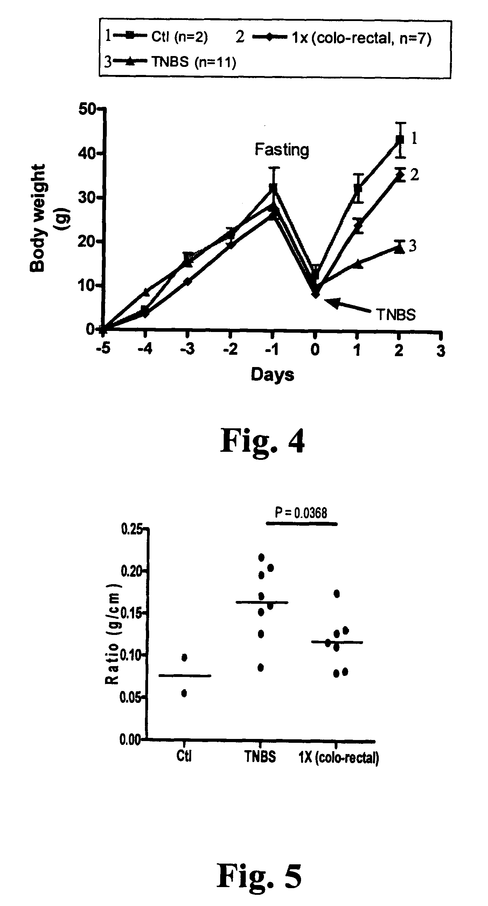

*Body weight.* As shown in Fig. 4, body weights of the animal increased constantly prior to fasting and TNBS injection in all groups. Following PBS injection (for control animals only), animals regained the weight lost during the fasting period. In TNBS-treated animals, however, the animals did not regain the body weight lost during the fasting period. As for the animals treated with the composition (1x) produced in example I and described in example II, body weight increased following the TNBS injection.

*Weight-to-length ratio.* TNBS injection increased weight-to-length ratio in colon for both TNBS and the composition (1x) produced in example I and described in example II groups, indicating the presence of inflammation and edema. However, the increase was significantly (P< 0.05) inferior in animals treated with the composition produced in example I and described in example II than in TNBS-treated animals (Fig. 5).

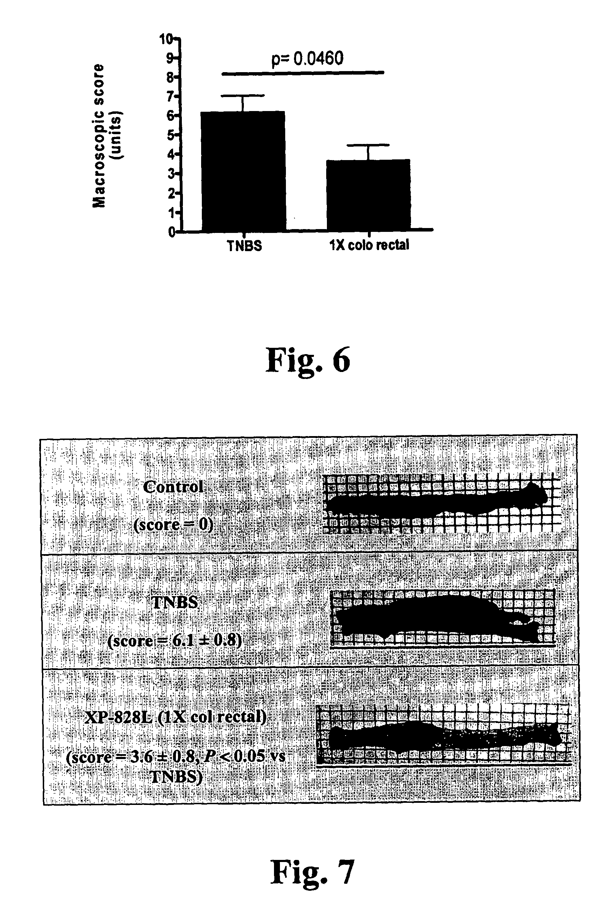

*Macroscopic* scores. TNBS injection increased macroscopic scores of inflammation in both groups treated with either TNBS and the composition (1x) produced in example I and described in example II, indicating the presence of inflammation and edema. However, the increase was significantly inferior (P< 0.05) in animals treated with the composition (1x) than in TNBS-treated animals (Fig. 6 and Fig. 7).

### EXAMPLE VI

### HLA-B27 rats

HLA-B27 transgenic rat, expressing *HLA-B27* and human β*2m,* exhibits a phenotype similar to humans suffering B27 related rheumatic disorders such as reactive arthritis, ankylosing spondylitis, psoriasis and inflammatory bowel disease (IBD). This rat model is useful for research and pharmacological studies of spontaneous systemic inflammation, arthritis, inflammatory bowel diseases and skin diseases including psoriasis and alopecia.

The primary goal of this experiement was to evaluate the *in vivo* efficacy of the composition (XP-828L) produced in example I and described in example II in IBD present in HLA-B27 rat model. Basically, four group of ten (10) male HLA-B27 transgenic rats (7 to 20 weeks old) were formed following randomization, which was based on the week of first symptoms of IBD (i.e. positive Hemoccult™). After one week of adaptation, the composition produced in example I and described in example II (7.82, 78.2 and 782 mg/kg) was orally administered daily via a gastric needle (total volume of 1.5 ml).

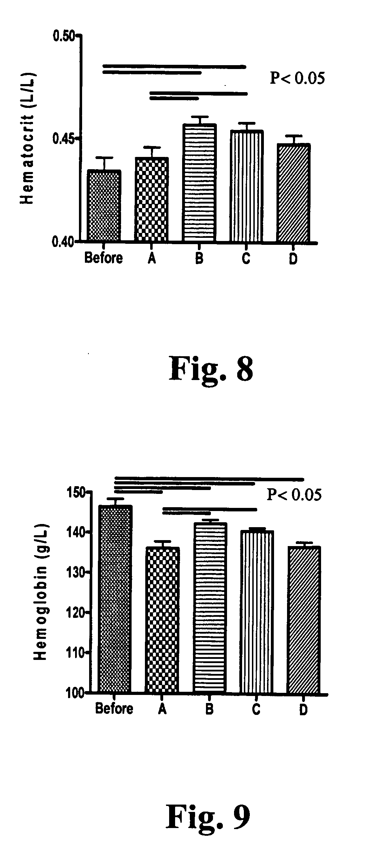

*Hematocrit.* As shown in Fig. 8, hematocrit levels were higher in rats treated with 7.82 (B) and 78.2 mg/kg (C) of the composition produced in example I and described in example II compared to the value observed before the beginning of the treatment. Also, animals treated with 7.82 and 78.2 mg/kg of the composition had a higher level of hematocrit compared to placebo (PBS) (A).

*Hemoglobin.* During the course of the disease, hemoglobin levels decreased significantly in all groups. However, the decrease was significantly lower in rats treated with 7.82 (B) and 78.2 mg/kg (C) of the composition produced in example I and described in example II compared to placebo (A) or even to 782 mg/kg (D) of the composition (Fig. 9).

### EXAMPLE VII

### Randomized double-blind, placebo-controlled study in humans

A randomized, double-blind, placebo-controlled study was undertaken to confirm the efficacy and safety of XP-828L in the treatment of mild to moderate psoriasis in human patients *(Poulin Y et al. (2006) XP-828L in the treatment of mild to moderate psoriasis: randomized, double-blind, placebo-controlled study. J. Cutan. Med. Surg).*

### Efficacy of XP-828L on psoriasis

Intra-group analyses on efficacy parameters are shown in Table 2. The most significant improvement in patients treated with XP-828L was seen in PGA scores, which decreased significantly by 0.3 ± 0.6 units (8.8 ± 17.2%, *p* < 0.05) from day 1 to day 56. This improvement was maintained at day 112 (0.3 ± 0.5 units or 8.7 ± 18.4%, *p* < 0.05). Additional analyses showed that few significant interaction effects were detected in PGA scores in age and gender. However, patients over 50 years old reported a higher PGA score than younger patients at day 1 and day 112 *(p* < 0.05) (data not shown).

BSA scores also improved in patients treated with XP-828L (Table 2). From day 1, BSA score improved by 0.5 ± 1.7 units (5.9 ± 17.3%, *p* <0.05) at day 56. The improvement was maintained at day 112. Significant differences in BSA score between genders (data not shown) were detected for day 1 (*p* = 0.006), day 56 (*p* = 0.0016) and day 112 (*p* = 0.017), with men having a higher BSA score on average. Ten patients (24%) improved their BSA score by 25% and higher from their initial score and maintained this improvement under XP-828L. No significant differences were observed with age concerning the patients treated with XP-828L (data not shown).

PASI score improved from day 1 to day 56 (p < 0.05). Eleven patients (26%) improved their PASI score by 25% and higher from initial score and maintained this improvement under XP-828L. On an individual basis, 5 patients out of 42 (11.9%) improved their PASI score by ≥ 40% in 56 days, while 8 patients out of 42 (19%) improved their PASI score by ≥ 40% during the entire study. PASI 50 was attained in 4 patients at day 56 and 6 patients at day 112. As for BSA, males reported a higher PASI score (data not shown) compared to female at each visit (Day 1, *p* = 0.009; Day 56, *p* = 0.020; Day 112, *p* = 0.022). Itching sensation decreased significantly at day 112 for patients taking XP-828L (*p* < 0.05) compared to day 1.

**Table 2. Efficacy of the XP-828L at day 1, 56 and 112, as evaluated by different assessment tools.**

| | **PASI** | **PGA** | **BSA** | **Itch severity** |
|---|---|---|---|---|
| | | | | |
| **Day 1** | 8.8±3.9 | 3.0±0.4 | 9.07±4.32 | 1.6±0.9 |
| **Day 56** | 8.3±4.3* | 2.8±0.6* | 8.65±4.78 | 1.3±0.9 |
| Δ Abs. | -0.5±1.8 | -0.3±0.6* | -0.5±1.7 | -0.2±0.9 |
| Δ % | -5.8±23.9 | -8.8±17.2* | -5.9±17.3* | -5.1±63.8 |
| **Day 112** | 8.3±4.4 | 2.8±0.7 | 8.66±5.53 | 1.3±0.9* |
| Δ Abs. | -0.5±2.5 | -0.3±0.5* | -0.4±3.5 | -0.3±0.8* |
| Δ % | -4.9±32.0 | -8.7±18.4* | -6.7±33.2 | -12.0±55.9 |

| | | | | |
|---|---|---|---|---|
| **Note:** Data are mean ± SD of 42 human subjects. * *p* < 0.05 compared to day 1. Δ Abs, absolute variation; Δ %, percent variation; PASI, psoriasis area and severity index; PGA, physician's global assessment; BSA, body surface area. | | | | |

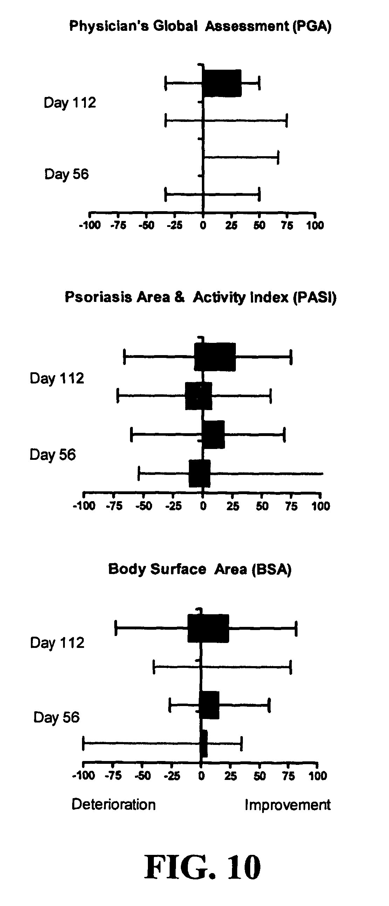

Relative improvements of the study endpoints are illustrated in Figure 10. As demonstrated, the vast majority of the patients improved all psoriasis assessment parameters from baseline at day 56 and day 112 indicating that patients could benefit from further improvement with longer use of XP-828L. PASI was improved as much as 25% in 8 patients at day 56 and in 11 patients at day 112. However, 2 patients show deterioration (> 25%) at day 56 and 3 at day 112. Three patients improved their BSA score by 25% at day 56 and 6 at day 112, while no deterioration was seen at day 56 and only one at day 112. PGA also was improved in 9 and 12 patients at days 56 and 112, respectively, while only 2 patients showed deterioration at day 112, compared to baseline.

### Prospective

Data available today suggest that XP-828L could modulate the inflammatory cascade seen in psoriasis through its systemic action on immune cells, mainly on T-lymphocytes. The proposed mechanism of action of XP-828L could also suggest a potential for the product to modulate in a beneficial way the immune system for the treatment or prevention of other immune-related diseases or other diseases related to an uncontrolled immune response like eczema and inflammatory bowel diseases.

### EXAMPLE VIII

### Process for the preparation of a Master-batch of XP-828L

➢ Rehydration: 2025 kg of WPI (whey protein isolate) from Armor Protéines, S.A.S., France (Protarmor 865; lot 06255*) is diluted in 41,000 L of water and cooled to 4°C (max 10°C). The mixture was homogenized for about 1 - 1.5 hour. *Preferably, the WPI lot chosen should contain a minimum of 1.8 µg of TGF-β2 /g of total protein having been subjected to a minimum of heat-treatment; the mixture was then left to stand at less than 10°C at pH 6.5 for 14 hours;
➢ Acidification: the pH of the solution was then adjusted to 5.1 ± 0.1 with HCl (Novacid, 34%) in a period of 2-3 hours;
➢ Heating: the solution was heated to 38°C by passing it through a heat exchanger at a rate of 10,500 U hour for about 4.5 hours;
➢ Centrifugation: the solution is centrifuged continuously in a Westfalia MSE300 separator at a rate of 11000 L / hour at 38°C;
➢ Dilution: the recovered precipitate was diluted in 11,000L of acidified water (at pH 5.1) containing 1.5 mM calcium chloride and 1.5 mM sodium chloride to maintain precipitate integrity. This suspension was agitated for 23 hours and then centrifuged again at 11,000 L/hour at 38°C;
➢ Neutralization: the precipitate was then neutralized to pH 6.92 with 12L of 15% NaOH; pasteurized 15 seconds at 75°C; ultrafiltrated; and then Spray-dried for 28 hours in a Cyclone Drying Tower (AP5).

The preparation yielded 103.2 kg of dried material for which the analysis is disclosed in the following example.

### EXAMPLE IX

***Analysis* of *Master Batch:***

| Scorched particles (ADMI) | A |
|---|---|
| Appearance | powder |
| pH of a 5% (w/w) solution | 8.9 |
| Moisture % | 4.01 |
| Crude proteins % | 90.9 |
| Ash % | 2.2 |
| **Bacteriological assessment** | |
| Mesophile: total plate count /g | 460 |
| Thermophile: total plate count /g | 430 |
| enterobacteria /g | <10 |
| yeasts /g | <5 |
| moulds /g | <5 |
| Spores SR 37°C /g | 10 |
| Listeria monocytogenes /25g | absent |
| Staphylococcus Aureus /g | absent |
| Salmonella /25g | absent |

## Claims

1. A process for the preparation of a whey protein-derived composition enriched in TGF-β, comprising the steps of:
a) adjusting whey proteins to a concentration of between 2% and 10% (w/v);
b) precipitating said whey proteins by acid treatment to a pH between 4.5 to 6.0 at a temperature of between 20°C and 50°C;
c) recovering said precipitate at a temperature of between 20°C and 50°C by physical means selected from the group consisting of: settling, sedimentation, centrifugation, aggregation and dialysis; and
d) neutralizing said precipitate to a pH between 6.7 to 7.3.

2. The process according to claim 1, further comprising the step of:
e) pasteurizing said precipitate.

3. The process according to claim 2, further comprising the step of:
f) spray-drying said precipitate to obtain a powder.

4. The process according to claim 1, wherein in step a) said whey proteins are diluted to a concentration of between 3% and 8% in water, preferably of 5 % in water.

5. The process according to claim 1, wherein in step b) said acid precipitation of the solution is carried out at a pH of between 4.6 to 5.8, preferably at a pH of between 4.8 and 5.2.

6. The process according to claim 1, wherein in step b) said acid precipitation of the solution is carried out at a temperature of between 30°C and 40°C.

7. The process according to claim 1, wherein in step c) said recovery of the precipitate is carried out at a temperature of between 30°C and 40°C.

8. The process according to claim 1, wherein in step d) said neutralization is carried out to a pH from 6.7 to 7.3, preferably at a pH from 6.8 to 7.2, more preferably to pH 7.

9. The process according to claim 2, wherein in step e) said recovered precipitate is subjected to a short pasteurization of less than 2 minutes.

10. The process according to claim 9, wherein said pasteurization is carried out for less than 1 minute, preferably for less than 30 seconds.

11. The process according to claim 1, wherein in step f) said retentate is ultrafiltrated on membranes having molecular weight cut-off (MWCO) up to a maximum of 10 kDa.

12. A process for the preparation of a whey proteins-derived composition enriched in TGF-β according to claim 1, comprising the steps of:
a) adjusting whey protein to a concentration of 5% (w/w) in water;
b) precipitating said whey proteins by acid treatment to a pH of 5.1 at a temperature of 35°C;
c) recovering said precipitate by centrifugation; and
d) neutralizing said precipitate to a pH of 7.

13. The process according to claim 12, further comprising the step of:
e) pasteurizing said precipitate for 15 seconds at a temperature of 75°C.

14. The process according to claim 1, wherein in step c) said recovery of said precipitate is achieved by centrifugation.

15. The process according to claim 9 or 10, wherein said pasteurization is carried out at a temperature of between 70°C and 80°C, preferably of between 72 and 78, more preferably between 74 and 76.

## Patentansprüche

1. Verfahren zum Herstellen einer von Molkenprotein abgeleiteten TGF-β-angereicherten Zusammensetzung, wobei das Verfahren die folgenden Schritte umfasst:
a) Einstellen von Molkenproteinen auf eine Konzentration zwischen 2 % und 10 % (Gew./Vol.);
b) Präzipitieren der Molkenproteine durch Säurebehandlung auf einen pH-Wert zwischen 4,5 und 6,0 bei einer Temperatur zwischen 20 °C und 50 °C;
c) Rückgewinnen des Präzipitats bei einer Temperatur zwischen 20 °C und 50 °C über physikalische Mittel, ausgewählt aus der Gruppe bestehend aus: Absetzung, Ablagerung, Zentrifugation, Aggregation und Dialyse; und
d) Neutralisieren des Präzipitats auf einen pH-Wert zwischen 6,7 und 7,3.

2. Verfahren nach Anspruch 1, ferner umfassend den folgenden Schritt:
e) Pasteurisieren des Präzipitats.

3. Verfahren nach Anspruch 2, ferner umfassend den folgenden Schritt:
f) Sprühtrocknen des Präzipitats, um ein Pulver zu erhalten.

4. Verfahren nach Anspruch 1, wobei die Molkenproteine in Schritt a) auf eine Konzentration zwischen 3 % und 8 % in Wasser, vorzugsweise 5 % in Wasser, verdünnt werden.

5. Verfahren nach Anspruch 1, wobei die Säurepräzipitation der Lösung in Schritt b) bei einem pH-Wert zwischen 4,6 und 5,8, vorzugsweise bei einem pH-Wert zwischen 4,8 und 5,2, erfolgt.

6. Verfahren nach Anspruch 1, wobei die Säurepräzipitation der Lösung in Schritt b) bei einer Temperatur zwischen 30 °C und 40 °C erfolgt.

7. Verfahren nach Anspruch 1, wobei die Rückgewinnung des Präzipitats in Schritt c) bei einer Temperatur zwischen 30 °C und 40 °C erfolgt.

8. Verfahren nach Anspruch 1, wobei die Neutralisierung in Schritt d) auf einen pH-Wert von 6,7 bis 7,3, vorzugsweise auf einen pH-Wert von 6,8 bis 7,2, mehr bevorzugt auf einen pH-Wert von 7, erfolgt.

9. Verfahren nach Anspruch 2, wobei das rückgewonnene Präzipitat in Schritt e) einer kurzen Pasteurisation von weniger als 2 Minuten ausgesetzt wird.

10. Verfahren nach Anspruch 9, wobei die Pasteurisation für weniger als 1 Minute, vorzugsweise für weniger als 30 Sekunden, erfolgt.

11. Verfahren nach Anspruch 1, wobei das Retentat in Schritt f) auf Membranen mit einem Molecular Weight Cut-off (MWCO) von bis zu 10 kDa ultrafiltriert wird.

12. Verfahren zur Herstellung einer von Molkenprotein abgeleiteten TGF-β-angereicherten Zusammensetzung nach Anspruch 1, wobei das Verfahren die folgenden Schritte umfasst:
a) Einstellen von Molkenproteinen auf eine Konzentration von 5 % (Gew./Gew.);
b) Präzipitieren der Molkenproteine durch Säurebehandlung auf einen pH-Wert von 5,1 bei einer Temperatur von 35 °C;
c) Rückgewinnen des Präzipitats durch Zentrifugation; und
d) Neutralisieren des Präzipitats auf einen pH-Wert von 7.

13. Verfahren nach Anspruch 12, ferner umfassend den folgenden Schritt:
e) Pasteurisieren des Präzipitats für 15 Sekunden bei einer Temperatur von 75 °C.

14. Verfahren nach Anspruch 1, wobei die Rückgewinnung des Präzipitats in Schritt c) durch Zentrifugation erfolgt.

15. Verfahren nach Anspruch 9 oder 10, wobei die Pasteurisation bei einer Temperatur zwischen 70 °C und 80 °C, vorzugsweise zwischen 72 °C und 78 °C, mehr bevorzugt zwischen 74 °C und 76 °C, erfolgt.

## Revendications

1. Procédé de préparation d'une composition dérivée de protéines lactosériques enrichie en TGF-β, comprenant les étapes consistant à :
a) ajuster les protéines lactosériques à une concentration comprise entre 2 % et 10 % (p/v) ;
b) précipiter lesdites protéines lactosériques par un traitement acide à un pH compris entre 4,5 et 6,0 à une température comprise entre 20 °C et 50 °C ;
c) récupérer ledit précipité à une température comprise entre 20 °C et 50 °C par des moyens physiques choisis dans le groupe constitué par : la décantation, la sédimentation, la centrifugation, l'agrégation et la dialyse ; et
d) neutraliser ledit précipité à un pH compris entre 6,7 à 7,3.

2. Procédé selon la revendication 1, comprenant en outre l'étape consistant à :
e) pasteuriser ledit précipité.

3. Procédé selon la revendication 2, comprenant en outre l'étape consistant à :
f) sécher par atomisation ledit précipité pour obtenir une poudre.

4. Procédé selon la revendication 1, dans lequel dans l'étape a) lesdites protéines lactosériques sont diluées à une concentration comprise entre 3 % et 8 % dans l'eau, de préférence de 5 % dans l'eau.

5. Procédé selon la revendication 1, dans lequel dans l'étape b) ladite précipitation acide de la solution est réalisée à un pH compris entre 4,6 et 5,8, de préférence à un pH compris entre 4,8 et 5,2.

6. Procédé selon la revendication 1, dans lequel dans l'étape b) ladite précipitation acide de la solution est réalisée à une température comprise entre 30 °C et 40 °C.

7. Procédé selon la revendication 1, dans lequel dans l'étape c) ladite récupération du précipité est réalisée à une température comprise entre 30 °C et 40 °C.

8. Procédé selon la revendication 1, dans lequel dans l'étape d) ladite neutralisation est réalisée à un pH de 6,7 à 7,3, de préférence à un pH de 6,8 à 7,2, plus préférablement au pH de 7.

9. Procédé selon la revendication 2, dans lequel dans l'étape e) ledit précipité récupéré est soumis à une courte pasteurisation inférieure à 2 minutes.

10. Procédé selon la revendication 9, dans lequel ladite pasteurisation est réalisée pendant moins de 1 minute, de préférence pendant moins de 30 secondes.

11. Procédé selon la revendication 1, dans lequel dans l'étape f) ledit rétentat est ultrafiltré sur des membranes ayant un poids moléculaire seuil (PMS) d'un maximum de 10 kDa.

12. Procédé de préparation d'une composition dérivée de protéines lactosériques enrichie en TGF-β selon la revendication 1, comprenant les étapes consistant à :
a) ajuster les protéines lactosériques à une concentration de 5 % (p/p) dans l'eau ;
b) précipiter lesdites protéines lactosériques par un traitement acide à un pH de 5,1 à une température de 35 °C ;
c) récupérer ledit précipité par centrifugation ; et
d) neutraliser ledit précipité à un pH de 7.

13. Procédé selon la revendication 12, comprenant en outre l'étape consistant à :
e) pasteuriser ledit précipité pendant 15 secondes à une température de 75 °C.

14. Procédé selon la revendication 1, dans lequel dans l'étape c) ladite récupération dudit précipité est obtenue par centrifugation.

15. Procédé selon la revendication 9 ou 10, dans lequel ladite pasteurisation est réalisée à une température comprise entre 70 °C et 80 °C, de préférence comprise entre 72 et 78, plus préférablement entre 74 et 76.
